# EUROPÄISCHE PATENTSCHRIFT

(11) **EP 0 704 442 B1**
(45) Veröffentlichungstag und Bekanntmachung des Hinweises auf die Patenterteilung: **06.08.2003**
(21) Anmeldenummer: 95114640.6
(22) Anmeldetag: 18.09.1995
(51) Int. Cl.: C07D 323/06, B01J 19/24

(54) **Naturumlaufreaktor und Verwendung**
Natural circulation reactor and use
Réacteur à circulation naturelle et utilisation

(30) Priorität: 29.09.1994 DE 4434845
(43) Veröffentlichungstag der Anmeldung: 03.04.1996
(73) Patentinhaber: Ticona GmbH, 65451 Kelsterbach (DE)
(72) Erfinder: Arnold, Dieter, D-61462 Königstein (DE); Hierholzer, Bernhard, Dr., D-60385 Frankfurt (DE); Wloch, Hubert, Dl., D-65527 Niedernhausen (DE); Mück, Karl-Friedrich, Dr., D-65207 Wiesbaden (DE)

(56) Entgegenhaltungen:
- EP-A- 0 012 304
- EP-A- 0 017 067
- EP-A- 0 028 361
- DD-A- 146 148
- W. FOERST (HRSG.): "Ullmanns Encyklopädie der technischen Chemie, 3. Auflage, 1. Band: Chemischer Apparatebau und Verfahrenstechnik" 1951 , URBAN & SCHWARZENBERG , MÜNCHEN - BERLIN XP002018369 * Seiten 529-544, insbesondere Seiten 533-536 *

## Beschreibung

Die Erfindung betrifft einen Reaktionsbehälter mit innen angeordneten Verdampferelementen sowie dessen Verwendung bei der Produktion von linearen oder zyklischen Acetalen.

Reaktionsbehälter der genannten Art sind bekannt und finden in der chemischen Industrie in vielen Prozessen Verwendung.

Die innen angeordneten Verdampferelemente sind üblicherweise in der Form von Platten, Rohrschlangen, Rohrbündeln oder anderen Rohrkonstruktionen ausgeführt, wobei durch die Platten oder Rohre ein Wärmeträgermedium fließt, das Wärme über die Platten oder Rohrwand an ein Reaktionsmedium im Reaktionsbehälter abgibt oder von diesem aufnimmt. Ist für bestimmte Reaktionen eine intensive Durchmischung des Reaktionsmediums erforderlich, so genügt die Konvektion, die die genannten Rohrkonstruktionen erzeugen, häufig nicht und es muß das Reaktionsmedium zusätzlich mit mechanischen Mitteln durchgemischt werden, beispielsweise über ein Rührwerk oder über einen externen Umpump. Eine Apparatur mit einem derartigen externen Kreislauf ist aus der EP 0 012 304 bekannt, in der ein Verfahren und eine Apparatur zur kontinuierlichen Herstellung von Trioxan beschrieben sind.
Das Trioxan wird dabei aus wässerigem Formaldehyd in Gegenwart saurer Katalysatoren bei höheren Temperaturen gebildet und durch Destillation aus dem Reaktionsgemisch abgetrennt. Für die intensive Durchmischung des Reaktionsgemisches sorgt ein externer Zwangsumlauf.

Als Maß für die Effektivität dieses Zwangsumlaufs kann bei diesem Verfahren das sogenannte Umlaufverhältnis herangezogen werden, das definiert ist als das Verhältnis von umlaufender Flüssigkeitsmenge in kg/s zu verdampfter Flüssigkeitsmenge in kg/s. Bei der Produktion von Trioxan ist ein Umlaufverhältnis größer als 50 anzustreben, das bislang nur über einen solchen externen Zwangsumlauf erreicht werden konnte. Dafür ist jedoch die Installation von kosten- und wartungsintensiven Apparaturen, Pumpen und Rohrleitungen erforderlich, die zusätzlich Sicherheitsrisiken bergen.

In Ullmanns Encyklopädie der technischen Chemie, 3. Aufl., 1. Band, Seiten 529-544 werden mehrere Verdampfer beschrieben: Der Robert-Verdampfer (Seite 533) besitzt innenliegende Heizkörper (Verdampferelemente, Siederohre), für die eine Rohrlänge von 0,7 bis 2,5 m angegeben ist. Jedoch ist hierbei nichts über das Querschnittverhältnis gesagt. Ferner werden Verdampfer mit außenliegendem Heizkörper erwähnt (Seite 534), wohingegen die Verdampferelemente des Anmeldungsgegenstandes innerhalb des Reaktors liegen. Auch werden Zwangsumlaufverdampfer beschrieben (Seite 536, Abb. 835), die innenliegende Heizkörper besitzen, deren Höhe jedoch nicht angegeben wird.

Aufgabe der Erfindung ist es, einen Reaktor der eingangs genannten Art so zu verbesseren, daß auch ohne zusätzliche mechanische Mittel eine ausreichende Durchmischung des Reaktionsmediums möglich ist und einen weiteren Reaktor bereitzustellen, der für die Herstellung von Trioxan geeignet ist.

Gemäß der Erfindung geschieht dies dadurch, daß die Verdampferelemente, in denen sich das Heizmedium befindet, mindestens zwei Meter hoch sind, diese Vertikal in der Mitte des Reaktors in einem Winkel von zwischen 3° und 15° zu einander angeordnet sind, so daß der Abstand zwischen benachbarten Verdampferelementen von unten nach oben zunimmt, der freie Querschnitt zwischen den Verdampferelementen e0 bis 80 % des Gesamtquerschnitts beträgt und Bleche um die Verdampferelemente angeordnet sind, so daß im Reaktor eine Thermosiphonströmung erzeugt wird, wobei die aufsteigende Strömung durch die Bleche von der absteigenden Strömung getrennt wird.
In vorteilhaften Ausführungen der Erfindung sind die Verdampferelemente Thermobleche. In einer weiteren vorteilhaften Ausgestaltung sind die Verdampferelemente aus Stahl oder aus einem korrosionsbeständigen Metall bzw. einer korrosionsbeständigen Legierung gefertigt.
Die Oberfläche der Verdampferelemente ist vorteilhaft poliert.
Über den Verdampferelementen ist vorteilhaft ein kastenförmiger Aufsatz angeordnet, der besonders bevorzugt seitlich dicht mit den um die Verdampferelementen angeordneten Blechen abschließt.

Der Aufsatz schließt seitlich dicht mit den Blechen 9 ab, so daß sich im Betriebszustand außerhalb des Aufsatzes ein höherer Flüssigkeitsstand ausbilden kann als innerhalb. Die Höhe des Aufsatzes umfaßt vorzugsweise den Bereich von 0,1 bis 0,8 m. Durch die unterschiedlichen Flüssigkeitsstände und den dadurch bedingten Druckgradienten wird das Umlaufverhältnis nochmals gesteigert.

Der erfindungsgemäße Reaktionsbehälter nach Anspruch 1 eignet sich zur Herstellung von linearen oder zyklischen Acetalen, insbesondere von Trioxan, Dioxolan, Tetroxan, Methylal, Dioxyether, Diethylacetal und Dibutylacetal.

Mit dem erfindungsgemäßen Reaktor können Umlaufverhältnisse bis 250 erreicht werden, wobei gleichzeitig die Sicherheit und die Verfügbarkeit der Produktionsanlage erhöht, die Störanfälligkeit vermindert wird.

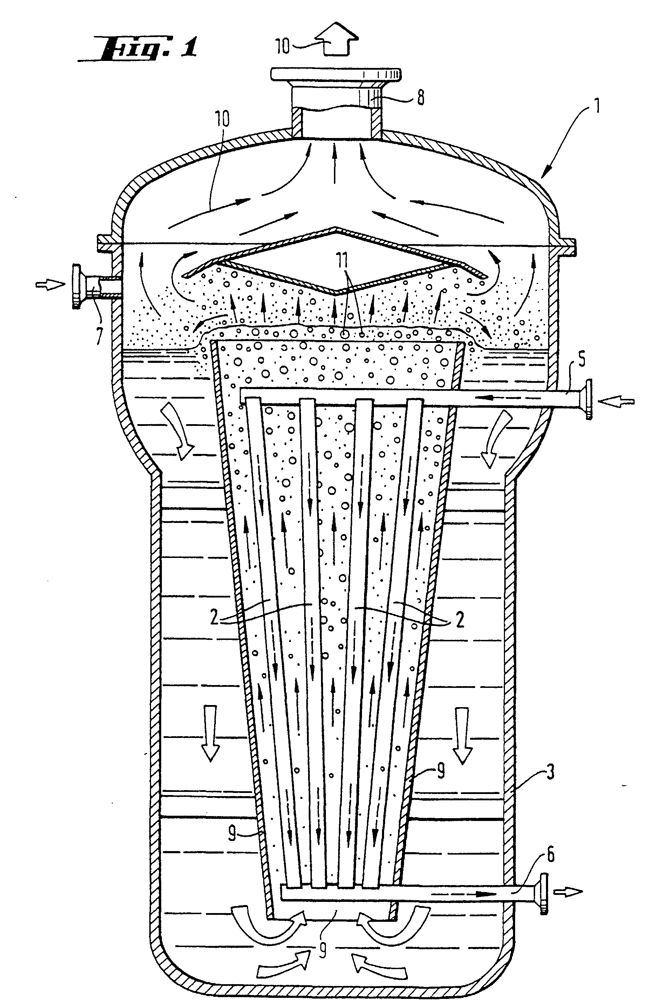

Im folgenden wird ein Ausführungsbeispiel der Erfindung anhand der Zeichnung näher beschrieben. Es zeigt
- Fig. 1: schematisch die Erfindung mit konisch zueinander angeordneten Verdampferelementen in Schnittansicht,

In einem in Fig. 1 dargestellten senkrecht stehendem Reaktionsbehälter 1 reagiert bei der Herstellung von Trioxan Formaldehyd in Anwesenheit von Säuren in einem flüssigen Medium 3 partiell zu Trioxan. Dieses Trioxan wird durch Verdampfen des Mediums 3 zu einem Dampf 10 gewonnen und mit dem Dampf 10 über einen Ausgang 8 abgezogen. Die Zugabe der Reaktionsedukte erfolgt über einen Zulauf 7.

Da sich Trioxan als Folge des Verdampfens in einer oberflächennahen Zone des Mediums 3 abreichert, muß, wenn eine möglichst hohe Trioxanausbeute erzielt werden soll, das Medium **3** intensiv durchmischt werden, damit die Trioxankonzentration in der oberflächennahen Zone möglichst nahe an der thermodynamischen Gleichgewichtskonzentration Dampf 10/Medium 3 liegt. Dies geschieht am besten dadurch, daß man das Medium 3 zirkulieren läßt.

Erfindungsgemäß wird dies dadurch erreicht, daß Verdampferelemente 2, die vorzugsweise als Platten ausgebildet sind, vertikal im Inneren des Reaktors angeordnet sind. Diese Platten 2 weisen Hohlräume auf, die von einem Wärmeträger, insbesondere Heizdampf, durchströmt werden können. Sie werden auch als Thermobleche bezeichnet und können bei Bedarf auch zum Kühlen verwendet werden. Der Eintritt des Wärmeträgers erfolgt über einen Zulauf 5, der Austritt durch einen Ablauf 6.

Werden die Platten 2 beheizt, so steigt das Medium 3 zwischen den Platten nach oben, unterstützt durch Dampfblasen, die sich je nach Verfahrensbedingungen bilden. Auf diese Weise wird gemäß dem Thermosiphonprinzip eine umlaufende Strömung - eine Thermosiphonströmung - erzeugt, die für die geforderte Zirkulation sorgt, wobei Umlaufverhältnisse größer 50 möglich sind. Dazu müssen die Platten 2 mindestens 2 m hoch sein und der freie Querschnitt zwischen den Platten 2 muß zwischen 20 und 80 %, vorzugsweise 30 bis 70 %, des Gesamtquerschnitts betragen, wobei der Gesamtquerschnitt definiert ist als Summe aus freiem Querschnitt zwischen den Platten und Querschnitt der Platten 2.

Der hydraulische Druckverlust, der von der Gesamtheit der Platten 2 verursacht wird, sollte in dem beschriebenen Beispiel 250 mbar nicht übersteigen.

Zur Trennung der aufsteigenden von der absteigenden Strömung sind Bleche 9 vertikal um die Platten 2 angeordnet. Bilden sich beim Heizen Dampfblasen 11 an den Oberflächen der Platte 2, so ist es vorteilhaft, daß die Platten konisch in einem vorgegebenen Winkel zwischen 3 und 15°, vorzugsweise zwischen 3 und 5°, zueinander angeordnet sind, wodurch sich der Abstand zwischen den Platten von unten nach oben vergrößert. Dadurch wird der hydraulische Widerstand des Raumes zwischen den Platten 2 von unten nach oben vermindert und so der Volumenvergrößerung des nach oben steigenden Dampf/Flüssigkeitsgemisches Rechnung getragen.

Weiterhin ist es vorteilhaft, die mediumsberührten Oberbeflächen der Platten 2 zu polieren, da dies den hydraulischen Widerstand ebenfalls vermindert.

Als Werkstoffe für Reaktor 1 und Platten 2 finden vorzugsweise Stahl oder rostund säurebeständige Stähle oder Sonderwerkstoffe Verwendung.

## Patentansprüche

1. Naturumlaufreaktor zur kontinuierlichen Herstellung von Trioxan mit innen nebeneinander angeordneten Verdampferelementen, **dadurch gekennzeichnet, daß** die Verdampferelemente, in denen sich das Heizmedium befindet, mindestens zwei Meter hoch sind, diese vertikal in der Mitte des Reaktors in einem Winkel von zwischen 3 und 15° zu einander angeordnet sind, so daß der Abstand zwischen benachbarten Verdampferelementen von unten nach oben zunimmt, der freie Querschnitt zwischen den Verdampferelementen 20 bis 80 % des Gesamtquerschnitts beträgt und Bleche um die Verdampferelemente angeordnet sind, so daß im Reaktor eine Thermosiphonströmung erzeugt wird, wobei die aufsteigende Strömung durch die Bleche von der absteigenden Strömung getrennt wird.

2. Reaktor nach Anspruch 1, **dadurch gekennzeichnet, daß** die Verdampferelemente Thermobleche sind.

3. Reaktor nach Anspruch 1, **dadurch gekennzeichnet, daß** die Verdampferelemente aus Stahl oder aus einem korrosionsbeständigen Metall oder aus einer korrosionsbeständigen Legierung gefertigt sind.

4. Reaktor nach Anspruch 1, **dadurch gekennzeichnet, daß** die äußere Oberfläche der Verdampferelemente poliert ist.

5. Reaktor nach Anspruch 1, **dadurch gekennzeichnet, daß** über den Verdampferelernenten ein kastenförmiger Aufsatz angeordnet ist.

6. Reaktor nach Anspruch 7. **dadurch gekennzeichnet, daß** der kastenförmige Aufsatz seitlich dicht mit den um die Verdampferelemente angeordneten Blechen abschließt.

7. Verwendung eines Reaktionsbehälters nach Anspruch 1 zur Herstellung von linearen oder zyklischen Acetalen, insbesondere Trioxan, Dioxolan, Tetroxan, Methylal, Dioxyether. Diethylacetal und Dibutylacetal.

## Claims

1. A natural circulation reactor for the continuous production of trioxane, which reactor has internally adjacently arranged evaporator elements, wherein the evaporator elements in which the heating medium is situated are at least two meters high, these are disposed vertically in the center of the reactor at an angle of between 3° and 15° to one another so that the distance between adjacent evaporator elements increases from bottom to top, the clear cross-sectional area between the evaporator elements is 20 to 80% of the overall cross-sectional area and sheets are disposed around the evaporator elements so that a thermosiphon flow is generated in the reactor, the ascending flow being separated from the descending flow by the sheets.

2. The reactor as claimed in claim 1, wherein the evaporator elements are heat-transfer sheets.

3. The reactor as claimed in claim 1, wherein the evaporator elements are fabricated from steel or from a corrosion-resistant metal or from a corrosion-resistant alloy.

4. The reactor as claimed in claim 1, wherein the outer surface of the evaporator elements is polished.

5. The reactor as claimed in claim 1, wherein a box-shaped attachment is arranged over the evaporator elements.

6. The reactor as claimed in claim 5, wherein the box-shaped attachment seals tightly with the sheets disposed around the evaporator elements.

7. The use of a reaction vessel as claimed in claim 1 for the production of linear or cyclic acetals, in particular trioxane, dioxolane, tetroxane, dimethoxymethane, diethoxymethane, diethoxyethane and dibutoxyethane.

## Revendications

1. Réacteur à circulation naturelle pour la production en continu de trioxane, comprenant des éléments d'évaporateur disposés à l'intérieur les uns à côté des autres, **caractérisé en ce que** les éléments d'évaporateur dans lesquels se trouve le milieu chauffant, sont au moins de deux mètres de haut, sont disposés verticalement au centre du réacteur suivant un angle compris entre 3 et 15° les uns par rapport aux autres, de sorte que la distance entre des éléments d'évaporateur voisins augmente de bas en haut, la section transversale libre entre les éléments d'évaporateur vaut 20 à 80% de la section transversale totale et des tôles sont prévues autour des éléments d'évaporateur de sorte qu'un écoulement par thermosiphon soit créé dans le réacteur, l'écoulement ascendant étant séparé par les tôles de l'écoulement descendant.

2. Réacteur selon la revendication 1, **caractérisé en ce que** les éléments d'évaporateur sont des tôles thermiques.

3. Réacteur selon la revendication 1, **caractérisé en ce que** les éléments d'évaporateur sont fabriqués en acier ou en un métal résistant à la corrosion ou en un alliage résistant à la corrosion.

4. Réacteur selon la revendication 1, **caractérisé en ce que** la surface extérieure des éléments d'évaporateur est polie.

5. Réacteur selon la revendication 1, **caractérisé en ce qu'**un couvercle en forme de caisse est disposé par-dessus les éléments d'évaporateur.

6. Réacteur selon la revendication 5, **caractérisé en ce que** le recouvrement en forme de caisse réalise une fermeture étanche latéralement avec les tôles disposées autour des éléments d'évaporateur.

7. Utilisation d'un réservoir de réacteur selon la revendication 1, pour la production d'acétals linéaires ou cycliques, en particulier du trioxane, du dioxolane, du tétroxane, du méthylal, du dioxyéther, du diéthylacétal et du dibutylacétal.
